# EUROPEAN PATENT APPLICATION

(11) **EP 4 644 900 A1**
(43) Date of publication of application: **05.11.2025**
(21) Application number: 24174152.9
(22) Date of filing: 03.05.2024
(51) Int. Cl.: G01N 33/543

(54) **QUALITY CONTROL OF BIOSENSORS**

(71) Applicant: QU-IP B.V., 1015 CS Amsterdam (NL)
(72) Inventor: Verheijden, Mark, 1015 CS Amsterdam (NL); Aphrham, Samer, 1015 CS Amsterdam (NL)
(74) Representative: Arnold & Siedsma

(57) **Abstract**

The present invention relates to a method for quality control of the ligand layer of a biosensor, comprising providing a biosensor coated with a ligand comprising a quality control target binding section and an analyte binding section and contacting the biosensor with a determined concentration of a quality control target molecule and determining the ligand density based on the binding signal generated by the quality control target molecule. The ligand is suitably bound to the sensor surface by the quality control target binding section of the ligand or by the analyte binding section of the ligand. The ligand can be a nucleic acid molecule, specifically a DNA probe. The ligand can also be a protein or an antibody. The method may comprise a further step of contacting the biosensor with the analyte to be detected.

## Description

The invention relates to a method for quality control of the ligand layer of a biosensor.

Different types of biosensors exist in the prior art. Some are based on physical or chemical phenomena, such as optical sensors, piezoelectric sensors or ion-selective biosensors. Others are based on binding of the analyte to a ligand or probe, such as biological molecules.

Biosensors for biological molecules are for example enzyme-based biosensors, immunoassay biosensors and DNA-based biosensors. Enzyme-based biosensors use enzymes as the biological recognition element. Enzymes catalyze specific reactions with the target analyte, and the resulting product is measured by the detector. Immunoassay biosensors utilize antibodies or antigens as the biological recognition element. They are commonly used in medical diagnostics for detecting various biomolecules such as hormones, proteins, and pathogens. DNA-based biosensors use DNA or RNA strands as the biological recognition element. They can detect specific DNA sequences or RNA molecules, making them useful for genetic analysis, disease diagnosis, and pathogen detection.

Early diagnosis facilitated by biosensors leads to timely treatment, improving patient outcomes and reducing healthcare costs. One of the significant advantages of biosensors is their potential for point-of-care (poc) testing. Such poc devices are portable and/or easy to operate and allow for immediate analysis of biological samples at the patient's bedside, in the doctor's office, or even in remote locations. This rapid testing capability is also very useful in emergency situations or in settings where access to laboratory facilities may be limited. Such biosensors may also be used at a patient's home for disease surveillance.

DNA biosensors, in particular surface-based DNA biosensors, have a high selectivity and sensitivity and are therefore very useful in the diagnosis and monitoring of several types of gene-related diseases, such as cancer.

Several strategies for probe immobilization on the sensor surface exist and are standardized, but reduced stability or affinity during hybridization may still occur. The probe density is a crucial parameter in biosensing because it affects the probe's orientation and the uniformity of the sensing layer. Moreover, the probe density not only provides possible binding sites for the analyte, but also affects the accessibility of the analyte toward the probe surface by the occurrence of steric and electrostatic effects.

A prerequisite for the reliability of the measurement by a biosensor is that the ligand layer is according to specification. The process of quality control (QC) is well-known in the field of biosensing, for instance for surface plasmon resonance (SPR) sensors. Techniques for testing the quality and density of the immobilized ligands (in this case DNA) include for example X-ray Photoelectron Spectroscopy (XPS), water contact angle (WCA), ellipsometry, and binding of the actual analyte and reading out with SPR or other sensing method used in specific applications. Another option is fluorescence labeling followed by fluorescence microscopy. For multiplexed sensors, one or more sensors can be functionalized with a reference ligand which target can be measured during the assay. Finally, a reference probe can be mixed in at a known ratio with the analyte probe during production.

This prior art quality control methods have a number of disadvantages, depending on the technique used. The QC analysis is often labor intensive and can only be performed as sampling. Potential variations, outliers, defects might be missed. Automation and testing of all sensors is potentially possible. However, this process is quite complex, expensive to set-up and the throughput might still be limited. In addition, the read-out is not quantitative so exact ligand densities cannot be derived. In addition to both points and related to all these techniques, the QC in the prior art is performed directly after production. Despite robustness and long-term stability testing, the DNA-ligands might degrade over time under certain conditions or if improperly stored. When using a reference ligand that is measured during the assay, the disadvantage is that one or more sensors have to be 'sacrificed' to the QC, rather than measuring an analyte molecule. In addition, the use of a separate reference ligand and real ligand can still result in errors if the reference is immobilized or stored correctly while the real ligand is not. When a reference probe can be mixed in at a known ratio, this can result in errors or variations during immobilization which will, unknowingly, influence the actually obtained ratio between reference and target probe. In addition, mixing different probes during the probe immobilization process, can make the immobilization step more complicated since the probes may interact with each other.

Therefore, there is need for a simplified and reliable method for the quality control of biosensors for biological molecules.

The present invention thus provides a method for quality control of the ligand layer of a biosensor, comprising:
a) providing a biosensor coated with a ligand comprising a quality control target binding section and an analyte binding section; and
b) contacting the biosensor with a determined concentration of a quality control target and determining the ligand density based on the binding signal generated by the quality control target.

The essence of the invention lies in the integration of two sections in one single molecule. Each ligand thus has its own integrated QC target.

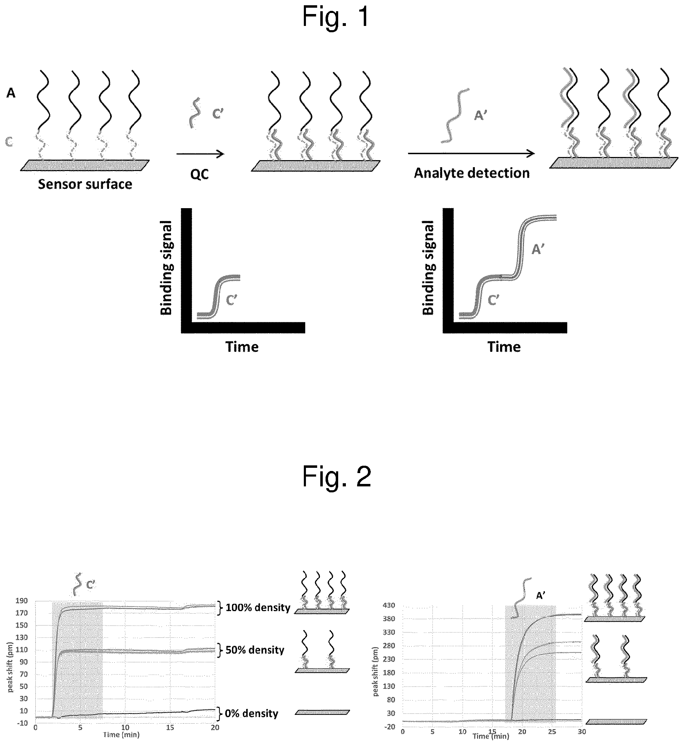

The invention is illustrated in **Figure 1**, showing the first section of the ligand molecule which is a quality control target binding section (C) that will bind a quality control target molecule (C'). The second section is the analyte binding section (A) that will bind the analyte (A') molecule for which the assay is designed.

By using one molecule that contains both the quality control target binding section and the analyte binding section, the quality control target binding step is intrinsically predictive of the probe density and presentation. During the assay, a known concentration of quality control target molecule (C') is introduced to the sensor which gives a pre-determined binding signal, indicating the sensor is within specifications. The binding process of sufficiently high concentrations of quality control target molecule C' results in fast (about 1 minute) saturation binding of the quality control target binding section.

The method of the invention thus provides an integrated process of quality control (QC) for the ligand layer in biosensors. This allows to perform a quality control step for the ligand layer of each individual biosensor, at the moment the (diagnostic) assay is performed. This way it becomes possible to check for each biosensor that is actually used in an assay whether it meets the specifications. There is no loss of the sensor and each sensor is tested.

According to the invention, the sensor is within specifications when the determined concentration of quality control target molecule generates a pre-determined binding signal.

In one embodiment, the quality control target binding section of the ligand is bound to the sensor surface. In another embodiment, the analyte binding section of the ligand is bound to the sensor surface.

In one embodiment, the ligand is a nucleic acid molecule, in particular a DNA probe. In a particular embodiment, the quality control target binding section is a DNA molecule that has no complementarity with any DNA expected in the target sample, which will avoid the non-specific binding of non-target DNA molecules that might be present in the sample.

In one embodiment, the ligand is a peptide or a protein such as an enzyme or antibody. In this case, the ligand can have a binding site other than the target binding site. This non-primary binding site can be used for the quality control step. The quality control is then performed by first or simultaneously contacting the biosensor with a molecule binding to the non-primary binding site, which is thus the quality control target binding section. The primary binding site is for binding the analyte.

When the ligand is an antibody, either two different antibodies can be used with different specificities or one bispecific antibody wherein one arm serves as the quality control target binding section and the other arm as the analyte binding section.

Also, the ligand may have functional handles, that are for example used in the purification of the ligand (such as a HIS-tag or Biotin-tag), which can also be used to elicit a complementary binding event for the QC step. The HIS-tag or biotin-tag are then the quality control target binding section, whereas another part of the protein is the analyte binding section. Finally, a second binding site could be engineered, similarly to the incorporation of a HIS-tag in a protein or peptide sequence.

The invention further relates to a method for testing a sample for the presence of an analyte, comprising performing the method as claimed in claim 1 and further comprising the step of
c) contacting the biosensor with the analyte to be detected.

When a biosensor with internal referencing is used, two distinct QC target sequences are designed. This way, matrix or other effects can be automatically subtracted from the binding signal. Some biosensors have an internal reference incorporated in the sensor design. This is the case for example for the asymmetric Mach Zehnder interferometer. These sensor designs automatically deduct matrix effects (salts, temperature changes) and non-specific binding events from the specific binding signal, leading to a more specific and stable signal. For optimal use of such design, both the target and the reference part of the sensor are functionalized with similar molecules, for example both with DNA probes. In this case, both target and reference part require a distinct QC section in order to be able to analyze both parts of the referenced sensor.

In the present application the following terminology is used. QC is the abbreviation of quality control. The term "probe" is used for a ligand comprising a DNA or RNA molecule. The "stem" is the quality control target binding section of the probe that binds the reference target.

The invention will be further elucidated in the Examples that follow, which are provided for illustration purposes and are not intended to limit the invention in any way.

In the specification and the Examples, reference is made to the following figures:
**Figure 1****:** Schematic picture of the invention. C is the section of the probe that will bind the quality control target C'. A is the section of the probe that will bind the analyte molecule A'.
**Figure 2****:** aMZI measurement of (LEFT) the binding of the synthetic QC target (C') to various QC-section containing probe densities and (RIGHT) the subsequent target DNA (A') binding on the same chip.
**Figure 3****:** aMZI measurement showing the effect of the addition of the Stem region to the probe. At high concentrations, a marginal effect is measured. At low (closer to realistic sample concentrations) concentration, no difference was observed.
**Figure 4****:** Correlation between QC target and main target binding, tested on different sensors, chips and for 2 chip coating types.
**Figure 5****:** Concentration-dependent binding of CO-StemA12, the reference target. Binding of CO-StemA12 at 100nM and 1uM.
**Figure 6****:** Performance of a second QC probe section by target binding analysis at 1uM.
**Figure 7****:** Binding and unbinding behavior of the QC target CO-StemA12 at 40°C in PBS buffer). The QC target is injected in the gray area, after which the buffer runs again over the sensors.
**Figure 8****:** comparing the location of the QC section in the probe: bottom and top.

### EXAMPLES

### EXAMPLE 1

### Method of the invention using an asymmetric Mach Zehnder interferometer (aMZI) chip

### Materials and design

The asymmetric Mach Zehnder interferometer (aMZI) chips with 6 sensors per chip used in these experiments were manufactured by LioniX International B.V. and a carboxylic acid terminated coating was applied by Surfix Diagnostics B.V. For readout, an optical readout system (OSROM) was used which was made by LioniX International B.V.

The probes used are called GHSR and CT47, which are sections of human genes. These are arbitrary sequences for illustrating the invention. Instead, the Stem/QC sections are designed artificially and have no complementarity with any DNA expected in the target sample which are not present in the human genome (concluded from BLAST search NIH NCBI). Many other non-human sequences of various lengths could be imagined/designed.

Probes and targets were ordered at IDT. The sequences of the various sections are listed in **Table 1.**

**Table 1**

| **Sequence name** | **Stem / QC section** | **Main analyte section** | **Functional group** |
|---|---|---|---|
| LP-23-GHSR-StemB 13 | AGAGAGAGAGAGA | TCGATCCAGTTCCATCTCGCACT | 3' Amine (/3AmMO/) |
| LP-CT47-Stem12A-TAM-Am | ACACACACACAC | CTTGTTCTTTCTCTTCCTCGCCTGC | /i5-TAMK/ and 3' Amine (/3AmMO/) |
| CO-Stem12A | GTGTGTGTGTGTTTT | - | |
| CO-StemB 13 | TCTCTCTCTCTCTTT | - | |

### Protocols

For DNA functionalization of the individual aMZI sensors on the chips, the following procedure was followed:
COOH coated chips were NHS activated by incubating the chip with a mixture of 200mM of EDC and 100mM NHS in 10mM MES buffer, freshly prepared. This activation was performed for 15 minutes. The surface was cleaned with MilliQ water using a squirt bottle and dried under a flow of nitrogen. The amine functional DNA probes were dissolved in 1.5M PB buffer to a concentration of 10uM. These probes were spotted using a Scienion^{™} spotter, for 1h at 70% humidity.

### Experimental results

### General concept

Multiple experiments have been performed showing the ability of the invention to provide fast (1-2 minutes) information on the probe density. The general concept and its performance are demonstrated in the experiment shown in **Figure 2****.**

### Effect of addition of QC target section

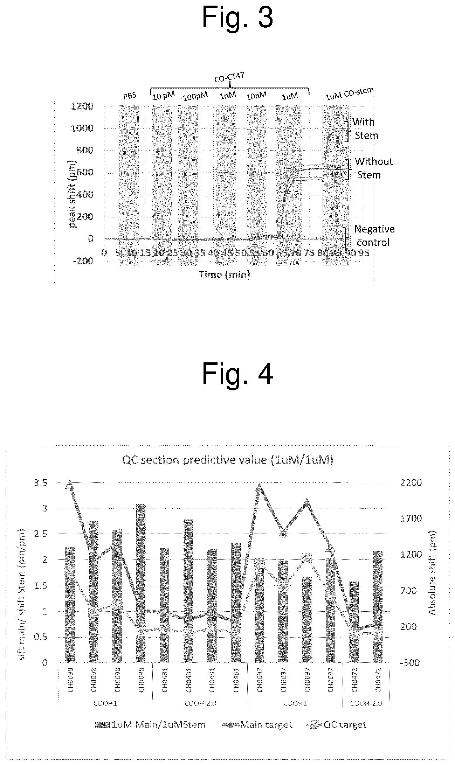

Initially, the effect of the addition of the QC (also named 'Stem') target section to the probe on the analyte binding was evaluated. This was done by using a probe with the QC target section and the same probe but without the QC target section on one biochip. The results showed a limited effect, meaning the QC target region does not interfere with analyte binding. This is shown in **Figure 3****.**

### Predicting analyte binding

The next step was to evaluate how well the QC target section and its reference target binding could 'predict' the binding of the analyte. This was done by always incorporating the QC target section in the analyte probe in all subsequent measurements. A selected set was analyzed, see **Figure 4****.** Despite significant variation in absolute binding values, a clear correlation was found between the QC target and the analyte binding if both were injected at high concentration (here 1 uM). This was observed for different sensors at one chip as well as among different chips.

### Determining optimal binding concentration

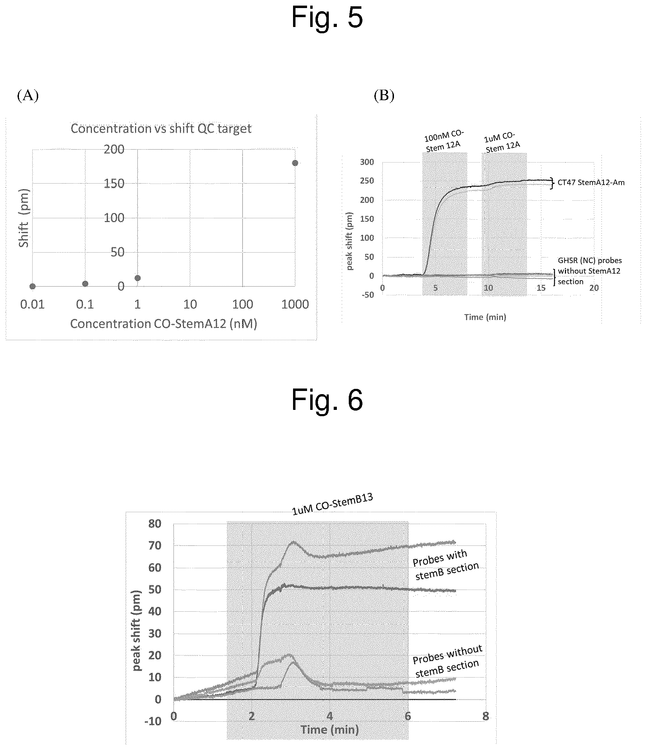

Next, it was evaluated at which target concentration the QC target would result in significant binding shift. Even though binding could be observed as low as 0.1 nM (see Figure 5), the most clear and fast binding is observed at 100nM or 1uM, at which concentrations the surface probes get saturated. This can be observed by the leveling-off of the binding curve at 100nM before the injection plug is finished, meaning the 100nM sample is still in contact with the sensor. In addition, it is clear from the fact that almost no additional binding is observed at an increased concentration of 1uM.

### Sensors with two sensing arms

The sensors we use have 2 sensing arms that together provide one net signal. One of the arms is meant for target measurement and one for reference. It would be most useful to be able to perform QC on both arms. Therefore, a second QC sequence was designed (StemB13) that can be used at this reference arm. The binding signal of this second QC sequence was lower than the first one, but still clearly visible (**Figure 6**). The lower signal is likely due to the lower density of this probe and not related to the QC target sequence.

### Temperature dependence

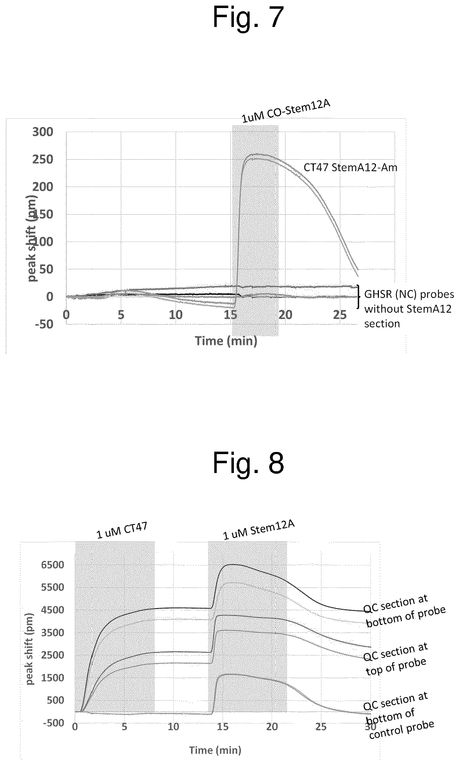

The above measurements were mostly performed at room temperature. If the assay temperature is increased, which is often done for DNA assays, the DNA-DNA interaction is weaker. This resulted in a reversible interaction of the CO-StemA12 (the QC-target) and the QC-probe section, as shown in **Figure 7****.** This is not unexpected considering the short length of the QC section: 12 bases. In fact, using the online tool MFold, the melting temperature of this complex can be predicted and is about 46°C, which is indeed close to the 40°C used in the measurement, explaining the reversible binding behavior. If irreversible behavior is desired to avoid having to wait for the unbinding process, the QC sequence can simply be chosen to be longer. Predicted melting temperatures for longer sequences are for example 46°C (12 bases), 53°C (14 bases), 58°C (16 bases). All these length changes, if desired, are minor design changes.

### Location of the QC target section

The QC target section can be incorporated at different places in the probe. Most obvious choices would be the bottom or the top. To not hinder the analyte binding, the bottom was selected for most experiments. The format with the QC target section at the top of the probe was also evaluated and this resulted in similar, albeit slightly faster QC target binding but also an undesired decrease in real analyte (here called CT47) binding, see **Figure 8****.**

### EXAMPLE 2

### Method of the invention using a Gold surface Qsense chips

### Materials and design

As an alternative to the aMZI chip used in Example 1, a Gold surface Qsense chips is used in these experiments (https://www.biolinscientific.com/qsense/sensors). First, a carboxylic acid termination is created with a carboxylate thiol by a method well known in the art.

The same probes and QC sections were used as in Example 1. The sequences of the various sections are listed in **Table 1.**

### Protocols

For thiol binding to the gold surface, the Gold surface QCM chips are first washed in piranha solution (a mixture of sulfuric acid (H₂SO₄) and hydrogen peroxide (H₂O₂)) and then washed with distilled water. The carboxylate thiol is bound to the gold surface by soaking it in a 10 mM thiol derivative dissolved in ethanol for 30 minutes. Afterwards, the gold surface is washed with ethanol, followed by distilled water, and dried under a flow of nitrogen.

For DNA functionalization of the carbonic acid groups on the surface, the same procedure was followed as in Example 1.

The same experiments were performed as described in Example 1 for the aMZI chip.

### Experimental results

In these experiments, the same results were obtained as with the aMZI chip in Example 1. It was thus found that also with this sensor, the binding of the QC target to the QC target binding section was representative of the ligand density on the sensor surface.

## Claims

1. Method for quality control of the ligand layer of a biosensor, comprising:
a) providing a biosensor coated with a ligand comprising a quality control target binding section and an analyte binding section; and
b) contacting the biosensor with a determined concentration of a quality control target molecule and determining the ligand density based on the binding signal generated by the quality control target molecule.

2. Method as claimed in claim 1, wherein the sensor is within specifications when the determined concentration of quality control target molecule generates a pre-determined binding signal.

3. Method as claimed in claim 1 or 2, wherein the quality control target binding section of the ligand is bound to the sensor surface.

4. Method as claimed in claim 1 or 2, wherein the analyte binding section of the ligand is bound to the sensor surface.

5. Method as claimed in claim 1 or 2, wherein the ligand is a nucleic acid molecule.

6. Method as claimed in claim 5, wherein the ligand is a DNA probe.

7. Method as claimed in any one of the claims 1 to 4, wherein the ligand is a protein, and the quality control target binding section and the analyte binding section have binding specificity for different molecules.

8. Method as claimed in any one of the claims 1 to 4, wherein the ligand is an antibody, wherein on arm serves are the quality control target binding section and the other arm serves as the analyte binding section, wherein the two arms have binding specificity for different antigens.

9. Method for testing a sample for the presence of an analyte, comprising performing the method as claimed in claim 1 and further comprising the step of:
c) contacting the biosensor with the analyte to be detected.
